# EUROPÄISCHE PATENTSCHRIFT

(11) **EP 3 755 730 B1**
(45) Veröffentlichungstag und Bekanntmachung des Hinweises auf die Patenterteilung: **04.09.2024**
(21) Anmeldenummer: 19703113.1
(22) Anmeldetag: 11.02.2019
(51) Int. Cl.: B01D 1/14, C08J 3/24, C08F 6/00, A61L 15/60, C08J 3/12, C08F 2/10, C08F 2/18

(54) **VERFAHREN ZUR HERSTELLUNG VON SUPERABSORBERPARTIKELN**
METHOD FOR PRODUCING SUPER ABSORBER PARTICLES
PROCÉDÉ DE PRODUCTION DE PARTICULES SUPERABSORBANTES

(30) Priorität: 22.02.2018 EP 18158090
(43) Veröffentlichungstag der Anmeldung: 30.12.2020
(73) Patentinhaber: BASF SE, 67056 Ludwigshafen am Rhein (DE)
(72) Erfinder: PFEIFFER, Thomas, 67056 Ludwigshafen (DE); WEISMANTEL, Matthias, 67056 Ludwigshafen (DE); SCHROEDER, Juergen, 67056 Ludwigshafen (DE); POSSEMIERS, Karl, 2040 Antwerpen (BE); KRUEGER, Marco, 67056 Ludwigshafen (DE); FUNK, Ruediger, 67056 Ludwigshafen (DE)
(74) Vertreter: BASF IP Association
(86) Internationale Anmeldenummer: PCT/EP2019/053280
(87) Internationale Veröffentlichungsnummer: WO 2019/162123

(56) Entgegenhaltungen:
- EP-A1- 2 927 264
- WO-A1-2017/207330

## Beschreibung

Die vorliegende Erfindung betrifft ein Verfahren zur Herstellung von Superabsorberpartikeln, umfassend Trocknung eines Polymergels, Abtrennung unvollständig getrockneter Polymerpartikel, Zerkleinerung der abgetrennten Polymerpartikel, Rückführung der zerkleinerten Polymerpartikel und Lagerung der rückgeführten Polymerpartikel.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Die Eigenschaften der Superabsorber können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Gelbettpermeabilität (GBP) und Absorption unter einem Druck von 49,2 g/cm² (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächen nachvernetzt. Dazu geeignete Vernetzer sind Verbindungen, die mit mindestens zwei Carboxylatgruppen der Polymerpartikel kovalente Bindungen bilden können.

EP 0 948 997 A2 offenbart ein Verfahren zur kontinuierlichen Herstellung von Superabsorbern, wobei unvollständig getrocknete Polymerpartikel abgetrennt und gegebenenfalls in die Trocknung zurückgeführt werden.

WO 2007/057350 A1 offenbart ein Verfahren zur kontinuierlichen Herstellung von Superabsorbern, wobei unvollständig getrocknete Polymerpartikel abgetrennt und zerkleinert werden.

WO 2017/207330 A1 beschreibt die Herstellung von Superabsorbern, lehrt aber keinen Zerkleinerungs- und auch keinen Lagerungsschritt für die unvollständig getrockneten Polymerpartikel.

Aufgabe der vorliegenden Erfindung war die Bereitstellung eines verbesserten Verfahrens zur kontinuierlichen Herstellung von Superabsorbern.

Gelöst wurde die Aufgabe durch ein Verfahren zur Herstellung von Superabsorberpartikeln, durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
umfassend die Schritte
i) Trocknung eines Polymergels zur Herstellung eines getrockneten Polymergels,
ii) Klassierung des getrockneten Polymergels aus Schritt i) unter Erhalt mindestens einer Fraktion unvollständig getrockneter Polymerpartikel und mindestens einer Fraktion getrockneter Polymerpartikel und
iii) Zerkleinerung der unvollständig getrockneten Polymerpartikel aus Schritt ii),
dadurch gekennzeichnet, dass die zerkleinerten Polymerpartikel aus Schritt iii) in das getrocknete Polymergel vor Schritt ii) oder in die getrockneten Polymerpartikel nach Schritt ii) rückgeführt und zusammen mit dem getrockneten Polymergel vor Schritt ii) bzw. den getrockneten Polymerpartikeln nach Schritt ii) in einem Behälter für einen Zeitraum von mindestens 5 Minuten gelagert werden.

In einer Ausführungsform der vorliegenden Erfindung werden die zerkleinerten Polymerpartikel aus Schritt iii) in das getrocknete Polymergel vor Schritt ii) rückgeführt und zusammen mit dem getrockneten Polymergel vor Schritt ii) in einem Behälter für einen Zeitraum von mindestens 5 Minuten gelagert. Anschließend können die getrockneten Polymerpartikel aus Schritt ii) mittels mindestens eines mehrstufigen Walzenstuhls gemahlen und mittels mindestens einer Taumelsiebmaschine klassiert werden.

In einer anderen Ausführungsform der vorliegenden Erfindung werden die zerkleinerten Polymerpartikel aus Schritt iii) in die getrockneten Polymerpartikel nach Schritt ii) rückgeführt und zusammen mit den getrockneten Polymerpartikeln nach Schritt ii) in einem Behälter für einen Zeitraum von mindestens 5 Minuten gelagert. Anschließend können die gelagerten Polymerpartikel mittels mindestens eines mehrstufigen Walzenstuhls gemahlen und mittels mindestens einer Taumelsiebmaschine klassiert werden.

Die gemahlenen und klassierten Polymerpartikel können anschließend thermisch Oberflächennachvernetzt werden.

Der Zeitraum für die Lagerung in dem Behälter beträgt vorzugsweise von 10 bis 240 Minuten, besonders bevorzugt von 20 bis 180 Minuten, ganz besonders bevorzugt von 30 bis 120 Minuten.

Der vorliegenden Erfindung liegt die Erkenntnis zugrunde, dass durch die gemeinsame Lagerung der zerkleinerten unvollständig getrockneten Polymerpartikel und Polymerpartikeln mit niedrigerer Feuchte zu einer Angleichung der Feuchte kommt. Durch Zerkleinerung und gemeinsame Lagerung können die unvollständig getrockneten Polymerpartikel ohne weitere Trocknung weiterverarbeitet werden.

Die in Schritt ii) erhaltenen getrockneten Polymerpartikel weisen einen Feuchtegehalt von vorzugsweise weniger als 10 Gew.-%, besonders bevorzugt von weniger als 7 Gew.-%, ganz besonders bevorzugt von weniger als 5 Gew.-%, auf. Der Feuchtegehalt kann durch die Trockenbedingungen eingestellt werden. Eine längere Trocknung bzw. höhere Trockentemperaturen führen zu einem niedrigeren Feuchtegehalt.

Die in Schritt ii) erhaltenen unvollständig getrockneten Polymerpartikel weisen einen Feuchtegehalt von vorzugsweise mindestens 5 Gew.-%, besonders bevorzugt mindestens 8 Gew.-%, ganz besonders bevorzugt mindestens 10 Gew.-%, höher als die in Schritt ii) erhaltenen getrockneten Polymerpartikel.

Die in Schritt ii) erhaltenen unvollständig getrockneten Polymerpartikel weisen eine Partikelgröße von vorzugsweise mindestens 8 mm, besonders bevorzugt mindestens 12 mm, ganz besonders bevorzugt mindestens 15 mm, auf. Zur Abtrennung der unvollständig getrockneten Polymerpartikel in Schritt ii) können alle zur Klassierung geeigneten Apparate eingesetzt werden, beispielsweise Siebe und Lochbleche. Die Klassierung kann auch so durchgeführt werden, dass mehr als eine Partikelgrößenfraktion unvollständig getrockneter Polymerpartikel und mehr als eine Partikelgrößenfraktion getrockneter Polymerpartikel anfallen.

In einer bevorzugten Ausführungsform der vorliegenden Erfindung werden zwei Partikelgrößenfraktionen unvollständig getrockneter Polymerpartikel erzeugt und nur die gröbere der beiden Partikelgrößenfraktionen in Schritt iii) zerkleinert. Die feinere der beiden Partikelgrößenfraktionen wird ohne Zerkleinerung in Schritt iii) rückgeführt.

In einer anderen Ausführungsform der vorliegenden Erfindung werden drei Partikelgrößenfraktion getrockneter Polymerpartikel erzeugt und nur die gröbere der drei Partikelgrößenfraktionen anschließend gemahlen und klassiert. Die mittlere der drei Partikelgrößenfraktionen weist bereits die gewünschte Partikelgröße auf und wird direkt mit den gemahlenen und klassierten Polymerpartikeln vereinigt. Die feinere der drei Partikelgrößenfraktionen wird in die Polymerisation oder das Polymergel vor der Trocknung rückgeführt.

Die in Schritt ii) erhaltenen unvollständig getrockneten Polymerpartikel werden in Schritt iii) auf eine Partikelgröße von vorzugsweise weniger als 15 mm, besonders bevorzugt weniger als 10 mm, ganz besonders bevorzugt weniger als 8 mm, zerkleinert. Es können alle zur Zerkleinerung geeigneten Apparate verwendet werden. Vorteilhaft werden Mühlen mit scharfen Schlägern oder Messern mit einem Sieb als Mahlraumbegrenzung eingesetzt. Die Maschenweite des Siebes beträgt vorzugsweise höchstens 15 mm, besonders bevorzugt höchstens 10 mm, ganz besonders bevorzugt höchstens 8 mm.

Die Behälter zur Lagerung unterliegen keiner Beschränkung. Geeignet sind beispielsweise Silos oder Vorlagebehälter. Für eine optimale Verteilung werden die rückgeführten Polymerpartikel vorteilhaft zu einem möglichst frühen Zeitpunkt der Zuführung der übrigen Polymerpartikel zum Behälter zugesetzt.

Die Polymerpartikel im Behälter weisen eine Temperatur von vorzugsweise 40 bis 120°C, besonders bevorzugt 50 bis 110°C, ganz besonders bevorzugt 60 bis 100°C, auf. Höhere Temperaturen begünstigen den Ausgleich des Feuchtegehalts, zu hohe Temperaturen schädigen die Polymerpartikel. Vorteilhaft wird der Behälter thermisch isoliert und/oder begleittbeheitzt.

Im Folgenden wird die Herstellung der Superabsorber näher erläutert:
Die Superabsorber werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessigsäure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite^{®} FF6 und Brüggolite^{®} FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.%, ganz besonders bevorzugt von 50 bis 65 Gew.-%. Es ist auch möglich Monomersuspensionen, d.h. Monomerlösungen mit die Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren für die Polymerisation sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Schritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen. Feste Carbonate und Hydrogencarbonate können hierbei auch in verkapselter Form eingesetzt werden, vorzugsweise in die Monomerlösung direkt vor der Polymerisation, während oder nach der Polymerisation ins Polymergel und vor dessen Trocknung. Die Verkapselung erfolgt durch Beschichtung der Oberfläche mit einem unlöslichen oder nur langsam löslichen Material (beispielsweise mittels filmbildender Polymere, inerter anorganischer Materialien oder schmelzbaren organischer Materialien), welches die Lösung und Reaktion des festen Carbonats oder Hydrogencarbonats so verzögert, dass erst während der Trocknung Kohlendioxid freigesetzt wird und der entstehende Superabsorber eine hohe innere Porosität aufweist.

Optional kann zur Monomerlösung vor oder während der Polymerisation ein Tensid zugegeben werden und die Monomerlösung dann vor oder während der Polymerisation mit einem Inertgas oder Wasserdampf oder durch starkes Rühren geschäumt werden. Das Tensid kann anionisch, kationisch, zwitterionisch oder auch nicht-ionisch sein. Bevorzugt wird ein hautfreundliches Tensid eingesetzt.

Das Polymergel wird in Schritt i) getrocknet. Der Feuchtegehalt nach der Trocknung beträgt vorzugsweise 0,5 bis 10 Gew.-%, besonders bevorzugt 1 bis 6 Gew.-%, ganz besonders bevorzugt 1,5 bis 4 Gew.-%, wobei der Feuchtegehalt gemäß der von der EDANA empfohlenen Testmethode Nr. WSP 230.2-05 "Mass Loss Upon Heating" bestimmt wird. Bei einer zu hohen Feuchte weist das getrocknete Polymergel eine zu niedrige Glasübergangstemperatur T_{g} auf und ist nur schwierig weiter zu verarbeiten. Bei einer zu niedrigen Feuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird in Schritt ii) klassiert. Dabei wird das getrocknete Polymergel in getrocknete Polymerpartikel und unvollständig getrocknete Polymerpartikel aufgetrennt.

Die unvollständig getrockneten Polymerpartikel werden in Schritt iii) zerkleinert. Die zerkleinerten unvollständig getrockneten Polymerpartikel werden rückgeführt.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150 bis 850 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Gelbettpermeabilität (GBP). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Es ist aber auch möglich die zu kleinen Polymerpartikel in einem dem Polymerisationsreaktor nachgeschalteten Schritt, beispielsweise in einem Kneter oder Extruder, in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen Polymerpartikel gesenkt. Dies kann aber beispielsweise durch Anpassung der Einsatzmenge an Vernetzer b) kompensiert werden.

Der Anteil an Polymerpartikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%.

Der Anteil an Polymerpartikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein. Zu große Polymerpartikel werden daher üblicherweise abgetrennt und in die Mahlung rückgeführt.

Die Polymerpartikel können zur weiteren Verbesserung der Eigenschaften thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 3 Gew.-%, besonders bevorzugt 0,02 bis 1 Gew.-%, ganz besonders bevorzugt 0,05 bis 0,2 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar^{®} Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix^{®} (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex^{®} Horizontal Paddle Dryer (Hosokawa Micron GmbH; Leingarten; Deutschland), Hosokawa Bepex^{®} Disc Dryer (Hosokawa Micron GmbH; Leingarten; Deutschland), Holo-Flite^{®} dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex^{®} Horizontal Paddle Cooler (Hosokawa Micron GmbH; Leingarten; Deutschland), Hosokawa Bepex^{®} Disc Cooler (Hosokawa Micron GmbH; Leingarten; Deutschland), Holo-Flite^{®} coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90°C, besonders bevorzugt 45 bis 80°C, ganz besonders bevorzugt 50 bis 70°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 40 bis 120°C, besonders bevorzugt bei 50 bis 110°C, ganz besonders bevorzugt bei 60 bis 100°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Oberflächennachvernetzung durchgeführt.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil^{®} 200, und Tenside, wie Span^{®} 20. Geeignete Beschichtungen zur Staubbindung, zur Verringerung der Verbackungsneigung sowie zur Erhöhung der mechanischen Stabilität sind Polymerdispersionen, wie in EP 0 703 265 B1 beschrieben, und Wachse, wie in US 5 840 321 beschrieben.

### Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategie Partners" EDANA (Avenue Eugène Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

### Feuchtegehalt

Der Feuchtegehalt der Superabsorberpartikel wird analog der von der EDANA empfohlenen Testmethode Nr. WSP 230.3 (11) "Mass Loss Upon Heating" bestimmt. Es wird eine 10fach-Bestimmung durchgeführt und das arithmetrische Mittel gebildet.

### Beispiele

### Beispiel 1

Eine zu 75 mol-% mit Natronlauge neutralisierte Acrylsäurelösung wurde zusammen mit Polyethylenglykoldiacrylat (Diacrylat eines Polyethylenglykols mit einem mittleren Molgewicht von 400 g/mol) in einem diskontinuierlichen Kneter polymerisiert. Das erhaltene krümelige Polymergel wurde bei 160°C in einem Umluftbandtrockner getrocknet. Das getrocknete Polymergel wurde noch im Bandtrockner mittels einer Stachelwalze grob zerkleinert (Polymerpartikel A).

Mittels eines Siebes mit einer Maschenweite von 10 mm wurden die unvollständig getrockneten Polymerpartikel (Polymerpartikel C) abgetrennt. Der Anteil der abgetrennten unvollständig getrockneten Polymerpartikel mit einem Partikeldurchmesser von größer 10 mm (Polymerpartikel C) betrug 5 Gew.-%. Die Polymerpartikel mit einer Partikelgröße von weniger als 10 mm wurden als getrocknete Polymerpartikel (Polymerpartikel B) weiterverarbeitet.

Der Feuchtegehalt der getrockneten Polymerpartikel (Polymerpartikel B) betrug 1,34 Gew.-% (Mehrfachbestimmung).

Die abgetrennten unvollständig getrockneten Polymerpartikel mit einem Partikeldurchmesser von größer 10mm (Polymerpartikel C) wurden händisch durch Schläge vom äußeren Trockenanteil befreit und mit einem Schneidwerkzeug auf einen Partikeldurchmesser von weniger als 10 mm zerkleinert.

Der Feuchtegehalt der zerkleinerten unvollständig getrockneten Polymerpartikel (Polymerpartikel D) betrug 9,73 Gew.-% (Mehrfachbestimmung).

122,5 g getrocknete Polymerpartikel (Polymerpartikel B) und 14,5 g zerkleinerte unvollständig getrocknete Polymerpartikel (Polymerpartikel D) wurden gemischt und in einer verschlossenen Glasflasche für einen Zeitraum von 30 Minuten bei 80°C gelagert.

Die gelagerte Mischung wurde dreistufig mit einem Walzenstuhl gemahlen. Die Mischung ließ sich problemlos verarbeiten.

Das Beispiel zeigt, dass es durch die gemeinsame Lagerung der getrockneten Polymerpartikel (Polymerpartikel B) und der zerkleinerten unvollständig getrockneten Polymerpartikel (Polymerpartikel D) auf eine zusätzliche Trocknung der unvollständig getrockneten Polymerpartikel verzichtet werden kann.

### Beispiel 2 (Vergleichsbeispiel)

Es wurde verfahren wie unter Beispiel 1. Die getrockneten Polymerpartikel (Polymerpartikel B) und die zerkleinerten unvollständig getrockneten Polymerpartikel (Polymerpartikel D) wurden gemischt und sofort dreistufig mit einem Walzenstuhl gemahlen. Die Mischung enthielt Polymerpartikel mit höherem Feuchtegehalt, die sich nicht mahlen ließen und beim Durchgang durch die Walzen zu Anbackungen und zu einer unerwüschten Belastung der Walzenlager führten.

### Beispiel 3

245 g mittels einer Stachelwalze grob zerkleinerter Polymerpartikel (Polymerpartikel A) und 29 g zerkleinerte unvollständig getrocknete Polymerpartikel (Polymerpartikel D), jeweils aus Beispiel 1, wurden in einer verschlossenen Glasflasche gemischt und für einen Zeitraum von 30 Minuten bei 80°C gelagert.

Mittels eines Siebes mit einer Maschenweite von 10 mm wurden die unvollständig getrockneten Polymerpartikel (Polymerpartikel C) aus der Mischung abgetrennt. Der Anteil der abgetrennten unvollständig getrockneten Polymerpartikel mit einem Partikeldurchmesser von größer 10 mm (Polymerpartikel C) betrug 4 Gew.-%.

Nach Abtrennung der unvollständig getrockneten Polymerpartikel (Polymerpartikel C) wurde die gelagerte Mischung sofort dreistufig mit einem Walzenstuhl gemahlen. Die Mischung ließ sich problemlos verarbeiten.

Das Beispiel zeigt, dass es durch die gemeinsame Lagerung der grob zerkleinerten Polymerpartikel (Polymerpartikel A) und der zerkleinerten unvollständig getrockneten Polymerpartikel (Polymerpartikel D) auf eine zusätzliche Trocknung der unvollständig getrockneten Polymerpartikel verzichtet werden kann.

### Beispiel 4 (Vergleichsbeispiel)

Es wurde verfahren wie unter Beispiel 3. Die grob zerkleinerten Polymerpartikel (Polymerpartikel A) und die zerkleinerten unvollständig getrockneten Polymerpartikel (Polymerpartikel D) wurden gemischt. Mittels eines Siebes mit einer Maschenweite von 10 mm wurden sofort die unvollständig getrockneten Polymerpartikel (Polymerpartikel C) aus der Mischung abgetrennt.

Nach Abtrennung der unvollständig getrockneten Polymerpartikel (Polymerpartikel C) wurde die Mischung sofort dreistufig mit einem Walzenstuhl gemahlen. Die Mischung enthielt Polymerpartikel mit höherem Feuchtegehalt, die sich nicht mahlen ließen und beim Durchgang durch die Walzen zu Anbackungen und zu einer unerwüschten Belastung der Walzenlager führten.

## Patentansprüche

1. Verfahren zur Herstellung von Superabsorberpartikeln, durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
umfassend die Schritte
i) Trocknung eines Polymergels zur Herstellung eines getrockneten Polymergels,
ii) Klassierung des getrockneten Polymergels aus Schritt i) unter Erhalt mindestens einer Fraktion unvollständig getrockneter Polymerpartikel und mindestens einer Fraktion getrockneter Polymerpartikel und
iii) Zerkleinerung der unvollständig getrockneten Polymerpartikel aus Schritt ii),
**dadurch gekennzeichnet, dass** die zerkleinerten Polymerpartikel aus Schritt iii) in das getrocknete Polymergel vor Schritt ii) oder in die getrockneten Polymerpartikel nach Schritt ii) rückgeführt und zusammen mit dem getrockneten Polymergel vor Schritt ii) bzw. den getrockneten Polymerpartikeln nach Schritt ii) in einem Behälter für einen Zeitraum von mindestens 5 Minuten gelagert werden.

2. Verfahren nach Anspruch 1, **dadurch gekennzeichnet, dass** die zerkleinerten Polymerpartikel aus Schritt iii) in das getrocknete Polymergel vor Schritt ii) rückgeführt und zusammen mit dem getrockneten Polymergel vor Schritt ii) in einem Behälter für einen Zeitraum von mindestens 5 Minuten gelagert werden.

3. Verfahren nach Anspruch 2, **dadurch gekennzeichnet, dass** die getrockneten Polymerpartikel aus Schritt ii) mittels mindestens eines mehrstufigen Walzenstuhls gemahlen und mittels mindestens einer Taumelsiebmaschine klassiert werden.

4. Verfahren nach Anspruch 1, **dadurch gekennzeichnet, dass** die zerkleinerten Polymerpartikel aus Schritt iii) in die getrockneten Polymerpartikel nach Schritt ii) rückgeführt und zusammen mit den getrockneten Polymerpartikeln nach Schritt ii) in einem Behälter für einen Zeitraum von mindestens 5 Minuten gelagert werden.

5. Verfahren nach Anspruch 4, **dadurch gekennzeichnet, dass** die gelagerten Polymerpartikel mittels mindestens eines mehrstufigen Walzenstuhls gemahlen und mittels mindestens einer Taumelsiebmaschine klassiert werden.

6. Verfahren nach einem der Ansprüche 3 oder 5, **dadurch gekennzeichnet, dass** die gemahlenen und klassierten Polymerpartikel thermisch Oberflächennachvernetzt werden.

7. Verfahren nach einem der Ansprüche 1 bis 6, **dadurch gekennzeichnet, dass** in dem Behälter für einen Zeitraum von 30 bis 120 Minuten gelagert wird.

8. Verfahren nach einem der Ansprüche 1 bis 7, **dadurch gekennzeichnet, dass** die in Schritt ii) erhaltenen getrockneten Polymerpartikel einen Feuchtegehalt von weniger als 5 Gew.-% aufweisen.

9. Verfahren nach einem der Ansprüche 1 bis 8, **dadurch gekennzeichnet, dass** die in Schritt ii) erhaltenen unvollständig getrockneten Polymerpartikel einen um mindestens 10 Gew.-% höheren Feuchtegehalt aufweisen als die in Schritt ii) erhaltenen getrockneten Polymerpartikel.

10. Verfahren nach einem der Ansprüche 1 bis 9, **dadurch gekennzeichnet, dass** die in Schritt ii) erhaltenen unvollständig getrockneten Polymerpartikel eine Partikelgröße von mindestens 8 mm aufweisen.

11. Verfahren nach einem der Ansprüche 1 bis 10, **dadurch gekennzeichnet, dass** die unvollständig getrockneten Polymerpartikel in Schritt iii) auf eine Partikelgröße von weniger als 8 mm zerkleinert werden.

12. Verfahren nach einem der Ansprüche 1 bis 11, **dadurch gekennzeichnet, dass** die unvollständig getrockneten Polymerpartikel in Schritt iii) mittels einer Mühle mit scharfen Schlägern oder Messern und einem Sieb als Mahlraumbegrenzung zerkleinert werden.

13. Verfahren nach einem der Ansprüche 1 bis 12, **dadurch gekennzeichnet, dass** die Polymerpartikel während der Lagerung im Behälter eine Temperatur im Bereich von 60 bis 100°C aufweisen.

14. Verfahren nach einem der Ansprüche 1 bis 13, **dadurch gekennzeichnet, dass** der zur Lagerung verwendete Behälter thermisch isoliert und/oder begleitbeheizt ist.

15. Verfahren nach einem der Ansprüche 1 bis 14, **dadurch gekennzeichnet, dass** ethylenisch ungesättigtes, säuregruppentragendes Monomer Acrylsäure ist.

## Claims

1. A process for producing superabsorbent particles by polymerizing a monomer solution or suspension comprising
a) at least one ethylenically unsaturated monomer which bears acid groups and may have been at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,
comprising the steps of
i) drying a polymer gel to produce a dried polymer gel,
ii) classifying the dried polymer gel from step i) to obtain at least one fraction of incompletely dried polymer particles and at least one fraction of dried polymer particles and
iii) comminuting the incompletely dried polymer particles from step ii),
wherein the comminuted polymer particles from step iii) are recycled into the dried polymer gel before step ii) or into the dried polymer particles after step ii) and stored together with the dried polymer gel before step ii) or the dried polymer particles after step ii) in a vessel for a period of at least 5 minutes.

2. The process according to claim 1, wherein the comminuted polymer particles from step iii) are recycled into the dried polymer gel before step ii) and stored together with the dried polymer gel before step ii) in a vessel for a period of at least 5 minutes.

3. The process according to claim 2, wherein the dried polymer particles from step ii) are ground by means of at least one multistage roll mill and classified by means of at least one tumbler screening machine.

4. The process according to claim 1, wherein the comminuted polymer particles from step iii) are recycled into the dried polymer particles after step ii) and stored together with the dried polymer particles after step ii) in a vessel for a period of at least 5 minutes.

5. The process according to claim 4, wherein the stored polymer particles are ground by means of at least one multistage roll mill and classified by means of at least one tumbler screening machine.

6. The process according to either of claims 3 and 5, wherein the ground and classified polymer particles are thermally surface postcrosslinked.

7. The process according to any of claims 1 to 6, wherein the storage period in the vessel is from 30 to 120 minutes.

8. The process according to any of claims 1 to 7, wherein the dried polymer particles obtained in step ii) have a moisture content of less than 5% by weight.

9. The process according to any of claims 1 to 8, wherein the incompletely dried polymer particles obtained in step ii) have a moisture content at least 10% by weight higher than the dried polymer particles obtained in step ii) .

10. The process according to any of claims 1 to 9, wherein the incompletely dried polymer particles obtained in step ii) have a particle size of at least 8 mm.

11. The process according to any of claims 1 to 10, wherein the incompletely dried polymer particles are comminuted in step iii) to a particle size of less than 8 mm.

12. The process according to any of claims 1 to 11, wherein the incompletely dried polymer particles are comminuted in step iii) by means of a mill with sharp beaters or blades and a screen as grinding space boundary.

13. The process according to any of claims 1 to 12, wherein the polymer particles have a temperature in the range from 60 to 100°C during the storage in the vessel.

14. The process according to any of claims 1 to 13, wherein the vessel used for storage is thermally insulated and/or trace heated.

15. The process according to any of claims 1 to 14, wherein ethylenically unsaturated monomer bearing acid groups is acrylic acid.

## Revendications

1. Procédé pour la production de particules superabsorbantes, par polymérisation d'une solution ou suspension de monomères contenant
a) au moins un monomère à insaturation éthylénique, portant des groupes acides, qui peut être au moins partiellement neutralisé,
b) au moins un agent de réticulation,
c) au moins un amorceur,
d) en option un ou plusieurs monomères à insaturation éthylénique copolymérisables avec les monomères cités en a) et
e) en option un ou plusieurs polymères hydrosolubles,
comprenant les étapes
i) séchage d'un gel polymère pour la production d'un gel polymère séché,
ii) classement du gel polymère séché provenant de l'étape i), avec obtention d'au moins une fraction de particules de polymère incomplètement séchées et d'au moins une fraction de particules de polymère séchées et
iii) fragmentation des particules de polymère incomplètement séchées provenant de l'étape ii),
**caractérisé en ce que** les particules de polymère fragmentées provenant de l'étape iii) sont renvoyées dans le gel polymère séché avant l'étape ii) ou dans les particules de polymère séchées après l'étape ii) et, conjointement avec le gel de polymère séché avant l'étape ii) ou avec les particules de polymère séchées après l'étape ii), stockées dans un récipient pendant une durée d'au moins 5 minutes.

2. Procédé selon la revendication 1,**caractérisé en ce que** les particules de polymère fragmentées provenant de l'étape iii) sont renvoyées dans le gel polymère séché avant l'étape ii) et, conjointement avec le gel de polymère séché avant l'étape ii), stockées dans un récipient pendant une durée d'au moins 5 minutes.

3. Procédé selon la revendication 2, **caractérisé en ce que** les particules de polymère provenant de l'étape ii) sont broyées au moyen d'au moins un broyeur à cylindres à plusieurs étages et classées au moyen d'au moins une machine tamiseuse oscillante.

4. Procédé selon la revendication 1, **caractérisé en ce que** les particules de polymère fragmentées provenant de l'étape iii) sont renvoyées dans les particules de polymère séchées après l'étape ii) et, conjointement avec les particules de polymère séchées après l'étape ii), stockées dans un récipient pendant une durée d'au moins 5 minutes.

5. Procédé selon la revendication 4, **caractérisé en ce que** les particules de polymère stockées sont broyées au moyen d'au moins un broyeur à cylindres à plusieurs étages et classées au moyen d'au moins une machine tamiseuse oscillante.

6. Procédé selon l'une quelconque des revendications 3 et 5, **caractérisé en ce que** les particules de polymère broyées et classées sont post-réticulées thermiquement en surface.

7. Procédé selon l'une quelconque des revendications 1 à 6, **caractérisé en ce que** le stockage dans le récipient est effectué pendant une durée de 30 à 120 minutes.

8. Procédé selon l'une quelconque des revendications 1 à 7, **caractérisé en ce que** les particules de polymère séchées, obtenues dans l'étape ii), présentent une teneur en humidité de moins de 5 % en poids.

9. Procédé selon l'une quelconque des revendications 1 à 8, **caractérisé en ce que** les particules de polymère incomplètement séchées, obtenues dans l'étape ii), présentent une teneur en humidité d'au moins 10 % en poids plus élevée que les particules de polymère séchées obtenues dans l'étape ii).

10. Procédé selon l'une quelconque des revendications 1 à 9, **caractérisé en ce que** les particules de polymère incomplètement séchées, obtenues dans l'étape ii), présentent une taille de particule d'au moins 8 mm.

11. Procédé selon l'une quelconque des revendications 1 à 10, **caractérisé en ce que** les particules de polymère incomplètement séchées sont fragmentées dans l'étape iii) jusqu'à une taille de particule de moins de 8 mm.

12. Procédé selon l'une quelconque des revendications 1 à 11, **caractérisé en ce que** les particules de polymère incomplètement séchées sont fragmentées dans l'étape iii) au moyen d'un broyeur à couteaux ou bras tranchants et comportant un tamis en tant que limite de chambre de broyage.

13. Procédé selon l'une quelconque des revendications 1 à 12, **caractérisé en ce que** les particules de polymère présentent pendant le stockage dans le récipient une température dans la plage de 60 à 100 °C.

14. Procédé selon l'une quelconque des revendications 1 à 13, **caractérisé en ce que** le récipient utilisé pour le stockage est isolé thermiquement et/ou doté d'un chauffage.

15. Procédé selon l'une quelconque des revendications 1 à 14, **caractérisé en ce que** le monomère à insaturation éthylénique, portant des groupes acides, est l'acide acrylique.
